# EUROPEAN PATENT APPLICATION

(11) **EP 2 030 557 A1**
(43) Date of publication of application: **04.03.2009**
(21) Application number: 08163372.9
(22) Date of filing: 01.09.2008
(51) Int. Cl.: A61B 1/005, A61B 1/01, A61B 1/273

(54) **Endoscopie guiding tube device**

(30) Priority: 31.08.2007 JP 2007224977; 31.08.2007 JP 2007224978; 31.08.2007 JP 2007224979
(71) Applicant: HOYA Corporation, Shinjuku-ku Tokyo 161-8525 (JP)
(72) Inventor: Ueda, Hirohisa, Shinjuku-ku Tokyo Tokyo 161-8525 (JP); Ohshima, Yuichi, Shinjuku-ku Tokyo Tokyo 161-8525 (JP); Ichikura, Shigeru, Shinjuku-ku Tokyo Tokyo 161-8525 (JP); Mukumoto, Toru, Shinjuku-ku Tokyo Tokyo 161-8525 (JP); Otsuka, Toshihiko, Shinjuku-ku Tokyo Tokyo 161-8525 (JP); Nakamura, Tetsuya, Shinjuku-ku Tokyo Tokyo 161-8525 (JP)
(74) Representative: Schaumburg, Thoenes, Thurn, Landskron

(57) **Abstract**

There is provided an endoscope guiding tube device including a flexible insertion tube, an endoscope guiding channel arranged in the flexible insertion tube along, at least one bending portion having a framework including a plurality of barrel-shaped joint rings coupled to each other in series to be rotatable with respect to each other, a plurality of shape-holding wires of which tip ends are attached to a tip portion of the at least one bending portion, and an operation unit in which proximal ends of the plurality of shape-holding wires are movable freely in an axial direction. The operation unit includes a wire locking mechanism having a function of locking together all of the proximal ends of the plurality of shape-holding wires and a function of releasing a locked state of all of the proximal ends of the plurality of shape-holding wires.

## Description

### Background of the Invention

The present invention relates to an endoscope guiding tube device inserted into a body cavity together with an insertion unit of a flexible endoscope to maintain the attitude and orientation of the insertion unit of the endoscope.

Conventionally, surgical operations for a target in an abdominal cavity (e.g., an operation for surgically removing a cholecyst) were conducted as an abdominal operation where an abdominal part of a patient is cut. However, recently, laparoscopically assisted surgery where a laparoscope (i.e., a rigid endoscope) or a surgical instrument is inserted into an abdominal cavity through a few holes opened in an abdominal part of a patient has become widespread. In general, each of such holes opened in an abdominal part of a patient has a size of a few centimeters. Therefore, if a patient undergoes laparoscopically assisted surgery, scars are left on a body surface in the abdominal part of the patient. It takes a certain time period for the patient to recover from the laparoscopically assisted surgery.

Recently, an endoscopic operation where an insertion unit of a flexible endoscope is inserted into a stomach from a mouth of a patient and a tip end of the insertion unit is passed through a hole formed through a wall of the stomach to conduct surgical treatment for a target in an abdominal cavity of the patient. In this case, a treatment instrument inserted into an instrument insertion channel of the flexible endoscope is used to open a hole through the wall of the stomach. After the endoscopic operation is finished, the hole is closed by an endoscopic clip.

Regarding such an endoscopic operation, it is difficult to maintain the attitude and orientation of the tip portion of the insertion unit of the flexible endoscope for a relatively long time during the endoscopic operation. That is, there is a possibility that the attitude and orientation of the tip portion of the insertion unit may change contrary to a surgeon's intention. Therefore, it is difficult to smoothly conduct the endoscopic operation for a target in an abdominal cavity.

Each of Published Japanese Translations of PCT international publication for a Patent Application No. JP 2006-512935A and Japanese Patent Provisional Publication No. JP 2005-318956A discloses an endoscope guiding tube device which assists insertion of an insertion unit of a flexible endoscope into a large intestine. However, an endoscope guiding tube device to be used for an endoscopic operation for a target in an abdomincal cavity has not been proposed.

### Summary of the Invention

The endoscope guiding tube device disclosed in each of JP 2006-512935A and JP 2005-318956A is configured to have a flexible insertion tube, in which an endoscope guiding channel is arranged, near an axis line, along the length of the flexible insertion tube. More specifically, the flexible insertion tube has a framework in which a plurality of saucer-shaped joint members, each of which has a through hole at the center thereof, are joined in series, and wires or a mesh tube are provided around the plurality of joint members. In this configuration, by drawing the wires (or the mesh tube) toward the proximal side, friction is produced between adjacent ones of the joint members and thereby the curved shape of the flexible insertion tube can be fixed.

However, such a structure of the endoscope guiding tube device requires a drawing mechanism for drawing strongly the wires (or the mesh tube). That is, a relatively large and sturdy drawing mechanism is required. Therefore, a size of the endoscope guiding tube device is inevitably increased. Further, such an endoscope guiding tube device has a drawback that when an external force acts on the joint members in the fixed shape, adjacent ones of the joint members may slide with respect to each other and thereby the fixed shape is changed. If each joint member is widened to increase friction to be produced between adjacent ones of the joint member while securing an adequate inner diameter of a guide channel, the entire size of the endoscope guiding tube device increases. In this case, the endoscope guiding tube device can not be inserted into a body cavity from a mouth of a person.

The present invention is advantageous in that it provides an endoscope guiding tube device which is capable of bending passively in accordance with change of the shape of an insertion unit of a flexible endoscope and maintaining stably the form in a desired shape and which is light and easy to use and exhibits a structural property that the diameter thereof can be reduced easily.

According to an aspect of the invention, there is provided an endoscope guiding tube device, which is provided with a flexible insertion tube, an endoscope guiding channel that is arranged in the flexible insertion tube along a length of the flexible insertion tube such that an insertion unit of an flexible endoscope can be inserted into the endoscope guiding channel, and at least one bending portion that forms the flexible insertion tube and has a framework including a plurality of barrel-shaped joint rings coupled to each other in series such that adjacent ones of the plurality of barrel-shaped joint rings are rotatable with respect to each other about a joint shaft provided between the adjacent ones of the plurality of barrel-shaped joint rings. The framework is deformable freely in all directions, the at least one bending portion is deformable passively in accordance with change in shape of the insertion unit of the flexible endoscope inserted into the endoscope guiding channel.

The endoscope guiding tube device is further provided with a plurality of shape-holding wires of which tip ends are attached to a tip portion of the at least one bending portion, and an operation unit provided at a proximal side of the flexible insertion tube and is configured such that proximal ends of the plurality of shape-holding wires are arranged in the operation unit to be movable freely in an axial direction of the flexible insertion tube. The operation unit includes a wire locking mechanism having a function of locking together all of the proximal ends of the plurality of shape-holding wires and a function of releasing a locked state of all of the proximal ends of the plurality of shape-holding wires.

With this configuration, the endoscope guiding tube channel is able to bend passively in accordance with change in shape of the insertion unit of the flexible endoscope, and is able to maintain its shape stably. It is not necessary to provide a drawing mechanism for the endoscope guiding tube device. The endoscope guiding tube device only requires a locking mechanism for locking the shape-holding wires. Therefore, an endoscope guiding tube device which is light and easy to use can be provided. Since the bending portion includes a plurality of barrel-shaped joint rings, it is possible to achieve a structural property that a diameter of the flexible insertion tube can be reduced easily. Consequently, it is possible to easily insert the endoscope guiding tube device into a mouth together with the insertion unit of the flexible endoscope.

In at least one aspect, the plurality of shape-holding wires include at least three shape-holding wires.

In at least one aspect, the at least three shape-holding wires are arranged about an axis line of the flexible insertion tube such that adjacent ones of the at least three shape-holding wires do not have an angular interval larger than or equal to 180 degrees.

In at least one aspect, the endoscope guiding channel has a flexible tube arranged near an axis line of the flexible insertion tube.

In at least one aspect, the endoscope guiding tube device further includes a sealing member provided at a proximal end of the endoscope guiding channel to seal a gap formed between an edge of an opening of the proximal end of the endoscope guiding channel and the insertion unit of the endoscope inserted into the endoscope guiding channel.

In at least one aspect, the endoscope guiding tube device further includes an outer sheath that has an elasticity and covers the at least one bending portion.

In at least one aspect, the at least one bending portion is provided on a front side of the flexible insertion tube, and a part of the flexible insertion tube other than the at least one bending portion is formed as a flexible tube part that is deformable passively in accordance with change in shape of the insertion unit of the flexible endoscope inserted into the flexible insertion tube, the flexible part being configured not to have a property of folding a shape thereof.

In at least one aspect, the wire locking mechanism includes a fixing base having a cone-shaped outer surface on which the proximal ends of the plurality of shape-holding wires move, a tapered cylinder that is arranged to be movable freely in the axial direction of the flexible insertion tube and has a tapered hole formed therein to form an inner surface facing the cone-shaped outer surface of the fixing base, and an operating member that moves the tapered cylinder in the axial direction. In this configuration, when the tapered cylinder is moved to a rear side by the operating member, the proximal ends of all of the plurality of shape-holding wires are sandwiched between the tapered cylinder and the fixing base and thereby the proximal ends of all of the plurality of shape-holding wires are locked. When the tapered cylinder is moved to a front side by the operating member, the proximal ends of all of the plurality of shape-holding wires become able to be movable in the axial direction, and thereby the at least one bending portion becomes able to be deformable passively in accordance with change in shape of the insertion unit of the flexible endoscope inserted into the endoscope guiding channel.

In at least one aspect, an elastic member is provided on the inner surface of the tapered cylinder.

In at least one aspect, each of the plurality of shape-holding wires is provided with a proximal mouthpiece at a proximal end portion thereof, and the proximal mouthpiece is locked between the tapered cylinder and the fixing base when the tapered cylinder is moved to the rear side.

In at least one aspect, a guiding groove is formed on the cone-shaped outer surface of the fixing base, the proximal mouthpiece of each of the plurality of shape-holding wires is guided, along the guiding grove, to be movable in the axial direction, and a depth of the guiding groove is smaller than a diameter of the proximal mouthpiece of each of the plurality of shape-holding wires.

In at least one aspect, a plurality of guide shafts is provided in the operation unit to guide the tapered cylinder to be movable in the axial direction.

In at least one aspect, the operation member includes an operation ring mounted in the operation unit to be rotatable about an axis line of the flexible insertion tube, a female thread being formed on the operation ring, the tapered cylinder is provided with a male thread such that the male thread engages with the female thread of the operation ring, and the tapered cylinder moves in the axial direction by rotating the operation ring about the axis line.

In at least one aspect, the endoscope guiding tube device further includes a plurality of flexible guide pipes of which tip ends are attached to a rear portion of the at least one bending portion, the plurality of shape-holding wires being respectively inserted into the plurality of flexible guide pipes to be movable in the axial direction. In this configuration, proximal ends of the plurality of flexible guide pipes are arranged in the operation unit to be movable in the axial direction, and the operation unit comprises a guide locking mechanism having a function of locking together all of the proximal ends of the plurality of flexible guide pipes and a function of releasing a locked state of all of the proximal ends of the plurality of flexible guide pipes.

In at least one aspect, each of the plurality of flexible guide pipes is formed of a coil pipe formed by tightly winding a metal wire to have a predetermined diameter.

In at least one aspect, the wire locking mechanism and the guide locking mechanism are formed as a single locking mechanism.

In at least one aspect, all of the proximal ends of the plurality of shape-holding wires and the plurality of flexible guide pipes are locked simultaneously by the single locking mechanism, and the locked state of all of the proximal ends of the plurality of shape-holding wires and the plurality of flexible guide pipes are released simultaneously by the single locking mechanism.

In at least one aspect, each of the plurality of flexible guide pipes is fixed in the operation unit.

In at least one aspect, the at least one bending portion comprises a plurality of bending portions coupled to each other in series, the tip ends of the plurality of flexible guide pipes are attached to rear portions of the plurality of bending portions, respectively, and the guide locking mechanism is able to lock together all of the proximal ends of the plurality of flexible guide pipes and is able to release the locked state of all of the proximal ends of the plurality of flexible guide pipes.

In at least one aspect, the guide locking mechanism includes a fixing base having a cone-shaped outer surface on which the proximal ends of the plurality of shape-holding wires move, a tapered cylinder that is arranged to be movable freely in the axial direction of the flexible insertion tube and has a tapered hole formed therein to form an inner surface facing the cone-shaped outer surface of the fixing base, and an operating member that moves the tapered cylinder in the axial direction. When the tapered cylinder is moved to a rear side by the operating member, the proximal ends of all of the plurality of guide pipes are sandwiched between the tapered cylinder and the fixing base and thereby the proximal ends of the plurality of guide pipes are locked. When the tapered cylinder is moved to a front side by the operating member, the proximal ends of the plurality of guide pipes become able to be movable in the axial direction.

In at least one aspect, each of the plurality of shape-holding wires is provided with a proximal mouthpiece at a proximal end portion thereof, and the proximal mouthpiece is locked between the tapered cylinder and the fixing base when the tapered cylinder is moved to the rear side.

In at least one aspect, the proximal mouthpiece has an outer diameter equal to an outer diameter of each of the proximal ends of the plurality of flexible guide pipes, and proximal mouthpieces of the plurality of shape-holding wires and the proximal ends of the plurality of flexible guide pipes are locked simultaneously between the tapered cylinder and the fixing base when the tapered cylinder is moved to the rear side.

In at least one aspect, a guiding groove is formed on the cone-shaped outer surface of the fixing base, the proximal end of each of the plurality of flexible guide pipes is guided, along the guiding groove, to be movable in the axial direction, and a depth of the guiding groove is smaller than a diameter of the proximal end of each of the plurality of flexible guide pipes.

In at least one aspect, the at least one bending portion comprises at least three bending portions coupled to each other in series.

### Brief Description of the Accompanying Drawings

Fig. 1 is a side view illustrating an entire configuration of an endoscope guiding tube device according to an embodiment of the invention.

Fig. 2 is a side cross section of a flexible insertion tube of the endoscope guiding tube device.

Fig. 3 is a partial side cross section of an endoscope guiding channel of the endoscope guiding tube device.

Fig. 4 illustrates a state where a framework of a bending portion of the endoscope guiding tube device is bent.

Fig. 5 is a cross section of the endoscope guiding tube device along a line V-V in Fig. 2.

Fig. 6 is a cross section of the endoscope guiding tube device along a line VI-VI in Fig. 2.

Fig. 7 is a cross section of the endoscope guiding tube device along a line VII-VII in Fig. 2.

Fig. 8 is a cross section of the endoscope guiding tube device along a line VIII-VIII in Fig. 2.

Fig. 9 is a side cross section of an operation unit of the endoscope guiding tube device.

Fig. 10 is a cross section of the endoscope guiding tube device along a line X-X in Fig. 9.

Fig. 11 is a side cross section of an operation unit of the endoscope guiding tube device, corresponding to state where shapes of bending portions are fixed.

Fig. 12 illustrates a situation where the endoscope guiding tube device is used for endoscopic treatment.

Fig. 13 illustrates a variation of an operation unit of an endoscope guiding tube device.

### Detailed Description of the Embodiments

Hereinafter, an embodiment according to the invention is described with reference to the accompanying drawings.

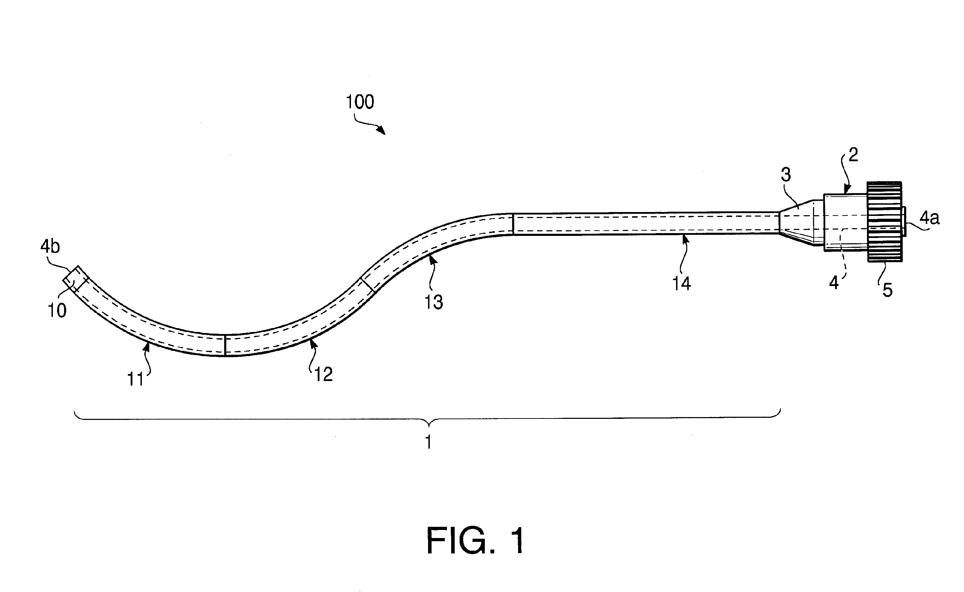

Fig. 1 illustrates an entire configuration of an endoscope guiding tube device 100 according to an embodiment of the invention. The endoscope guiding tube device 100 includes a flexible insertion tube 1 and an operation unit 2. The flexible insertion tube 1 is configured to be able to bend freely in all directions and can be inserted into a body cavity. The operation unit 2 is attached to a proximal end of the flexible insertion tube 1.

At a connection part between the operation unit 2 and the proximal end of the flexible insertion tube 1, a safety stopper 3 made of elastic material (e.g., rubber) is attached so as to prevent the flexible insertion tube 1 from buckling at the proximal end portion thereof. The safety stopper 3 has a tapered end.

The flexible insertion tube 1 has bending portions 11, 12 and 13 connected in series from a tip end thereof. Each of the bending portions 11, 12 and 13 is formed to be able to bend freely in all directions. At a proximal portion of the flexible insertion tube 1, a flexible tube part 14 is provided. The flexible tube part 14 is also formed to be able to bend freely in all directions.

More specifically, each of the bending portions 11, 12 and 13 is formed such that the shape of the bending portion can be fixed in a desired shape by operating an operation ring 5. On the other hand, the flexible tube part 14 is not able to fix its shape. The configurations of the bending portions 11, 12 and 13, and the flexible tube part 14 are described in detail later.

It should be noted that the flexible insertion tube 1 may be formed not to have the flexible tube part 14 depending on use of the endoscope guiding tube device 100. If the flexible insertion tube 1 is formed not to have the flexible tube part 14, the entire flexible insertion tube 1 may be formed as a bending portion. The number of bending portions is not limited to the example shown in Fig. 1. For example, the flexible insertion tube 1 may be formed of a single bending portion. Alternatively, the flexible insertion tube 1 may be formed of three or more bending portions. In another example, a relatively short flexible tube part may be provided between adjacent ones of the bending portions 11-13. In another example, a relatively short rigid tube part may be provided between adjacent ones of the bending portions 11-13. The type of the flexible insertion tube to be used may be selected from the above described variations depending on use of the endoscope guiding tube device 100.

In the flexible insertion tube 1, an endoscope guiding channel 4 is formed throughout the length of the flexible insertion tube 1. In the endoscope guiding channel 4, an insertion tube of a flexible endoscope can be inserted. A proximal end opening 4a of the endoscope guiding channel 4 is situated at a rear surface of the operation unit 2, and a tip end opening 4b of the endoscope guiding channel 4 is situated at a front surface of a mouth ring 10 provided at the tip end of the flexible insertion tube 1.

In this configuration, by inserting an insertion tube of a flexible endoscope into the endoscope guiding channel 4 from the proximal end opening 4a, it is possible to project or retract a tip end of the insertion tube of the flexible endoscope with respect to the tip end opening 4b of the endoscope guiding channel 4.

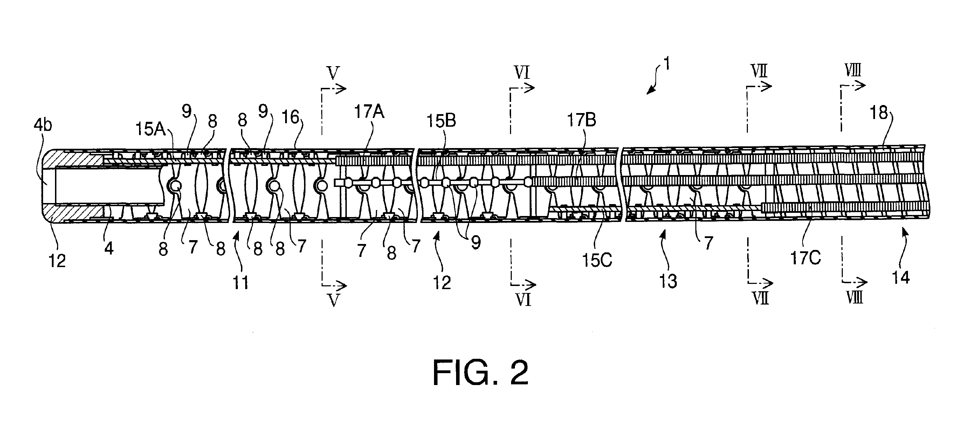

Fig. 2 is a side cross section of the flexible insertion tube 1. The mouth ring 10 which is made of metal or rigid plastic has a form of a ring. The mouth ring 10 is attached to the tip end of the bending portion 11. A flexible tube forming the endoscope guiding channel 4 is adhered to the rear part of the mouth ring 10 to secure a watertight property. The flexible tube is formed to be elastically bent together with the insertion tube of the flexible endoscope inserted thereto.

Although Fig. 2 illustrates only a front side of the flexible insertion tube 1, the flexible tube forming the endoscope guiding channel 4 is provided throughout the length of the flexible insertion tube 1. A proximal end of the flexible tube is situated in the operation unit 2.

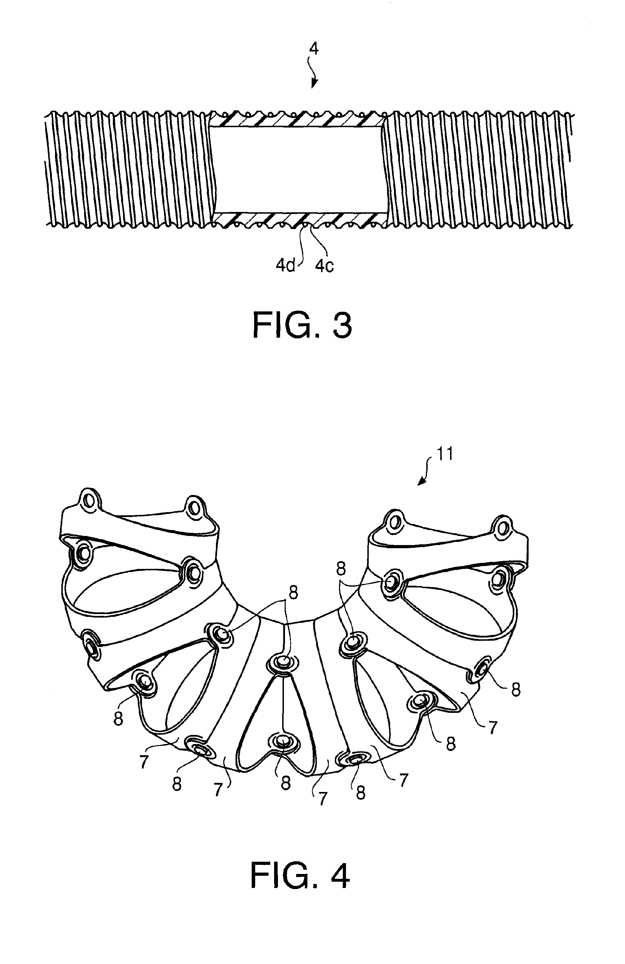

The flexible tube forming the endoscope guiding channel 4 may be provided with reinforcements to enhance a resistance property with respect to buckling. For example, a coil spring 4d may be wound along a spiral groove 4c formed around the outer surface of the flexible tube (see Fig. 3).

As shown in Fig. 2, the bending portion 11 has a framework including a plurality of barrel-shaped joint rings 7 coupled to each other. More specifically, adjacent ones of the joint rings 7 are coupled to be rotatable with respect to each other about a joint shaft 8 provided at tongue parts of the adjacent joint rings 7. With this configuration, the bending portion 11 is able to bend freely in all directions. Since a thickness of 0.3 mm to 0.4 mm is adequate for forming the joint ring 7, it is possible to maintain the outer diameter of the flexible insertion tube 1 at a small value.

Fig. 4 illustrates a state where the framework of the bending portion 11 is bent at the maximum. As shown in Fig. 4, the framework of the bending portion 11 is configured such that angular positions of the joint shafts 8 of the adjacent joint rings 7 shift by 90 degrees with respect to each other. However, another angular displacement (e.g., 60 degrees) may be employed for the angular positions of the joint shafts 8.

As shown in Fig. 2, along an inner surface of the foremost joint ring 7 of the bending portion 11, a tip end of a shape-holding wire 15a is fixed. A twisted wire formed of stainless steel may be used as the shape-holding wire 15a.

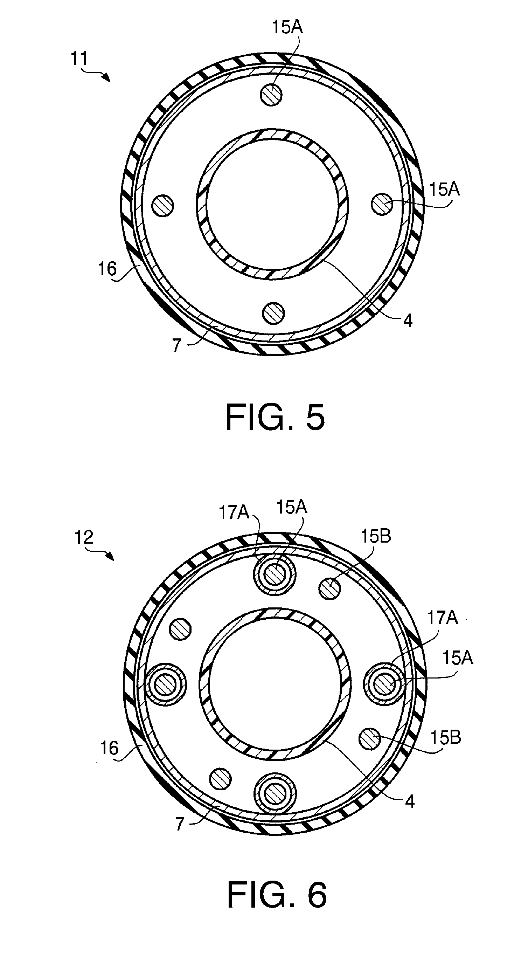

In the bending portion 11, the shape-holding wire 15a is inserted into wire guide holds 9 arranged at certain intervals on the inner surfaces of the joint rings 7. Each of the wire guide holes 9 is formed to protrude inwardly from the inner surface of the joint ring 7. It should be noted that, although in Fig. 2 only one shape-holding wire 15a is illustrated for the sake of simplicity, actually four shape-holding wires 15A are provided along the inner surfaces of the joint rings 7. More specifically, as shown in Fig. 5 which is a cross section of the bending portion 11, the shape-holding wires 15A are arranged at angular intervals of 90 degrees about an axis line of the bending portion 11. Each of the tip ends of the shape-holding wires 15A is fixed to the inner surface of the foremost joint ring 7.

As shown in Fig. 2, each of the shape-holding wires 15A is inserted into a flexible guide pipe 17A to be movable back and forth in the guide pipe 17A. A tip end of the guide pipe 17A is fixed to the inner surface of the bending portion 11 at a rear end of the bending portion 11. The guide pipe 17A is arranged in the flexible insertion tube 1 in parallel with the axis line of the flexible insertion tube 1.

More specifically, four guides pipes 17A are provided in the flexible insertion tube 1. Each of the four guide pipes 17A is arranged in the insertion tube 1 such that only the tip end thereof is fixed to one of components in the flexible insertion tube 1. Similar to the arrangement of the shape-holding wires 15A, the guide pipes 17A are arranged about the axis line of the bending portion 11 to have the angular intervals substantially equal to 90 degrees and to be in parallel with the axis line of the flexible insertion tube 1. A proximal end of the guide pipe 17A is situated in the operation unit 2.

For example, a coil pipe formed by tightly winding a metal wire (e.g., a stainless steel wire) to have a certain outer diameter may be employed as the guide pipe 17A. Another type of pipe exhibiting resistance to pressure in the axial direction may be employed as the guide pipe 17A.

The bending portion 11 is covered with an outer sheath tube 16 having flexibility and elasticity, such as a rubber tube, to achieve a watertight property. To prevent the outer sheath tube 16 from being nipped in a gap formed between adjacent joint rings 7, a mesh tube is provided on the inside of the outer sheath tube 16.

In this embodiment, the entire flexible insertion tube 1 is covered with one outer sheath tube 16 continuously formed along the length of the flexible insertion tube 1. However, in another example, separate outer sheath tubes may be provided respectively for the bending portions 11-13 and the flexible tube part 14. In this case, the bending portions 11-13 and the flexible tube part 14 are respectively covered with separate outer sheath tubes. In another example, only the flexible tube part 14 may be covered with a synthetic resin tube more rigid than a rubber tube.

The bending portion 11 formed as described above can be deformed freely in response to change of the shape of an insertion tube of a flexible endoscope inserted into the endoscope guiding channel 4 because the four shape-holding wires 15A are not fixed in the bending portion 11 excepting the tip ends thereof and thereby the shape-holding wires 15A are movable in the axial direction. In the state where all of the proximal ends of the shape-holding wires 15A and the guide pipes 17A are fixed in the operation unit 2, the bending portion 11 becomes unable to bend. That is, the shape of the bending portion 11 is fixed.

A required number of shape-holding wires 15A to provide the ability to hold the shape bending portion 11 in a desired shape for the bending portion 11 is three. That is, if the holding portion 11 is provided with at least three shape-holding wires 15A, the holding portion 11 is able to keep its shape in a desired shape. In this case, although the shape-holding wires 15A are not required to be arranged to have the same angular intervals, adjacent shape-holding wires 15A are required not to have an angular interval larger than or equal to 180 degrees.

When the four shape-holding wires 15A are provided as shown in Fig. 5, the shape-holding wires 15A are not required to be arranged to have the same angular intervals as long as adjacent shape-holding wires 15A are arranged not to have an angular interval larger than or equal to 180 degrees.

The bending portion 12 has substantially the same structure as that of the bending portion 11. That is, the bending portion 12 has a framework in which a plurality of joint rings 7 are coupled to each other in series such that adjacent ones of the joint ring 7 are rotatable with respect to each other about the joint shaft 8. With this structure, the bending portion 12 can be bent freely in all directions.

As shown in Fig. 2, a tip end of each shape-holding wire 15B is fixed to the inner surface of the foremost joint ring 7 in the bending portion 12. Flexible guide pipes 17B are provided in the bending portion 12 to be aligned with the shape-holding wires 15B, respectively. Each of the shape-holding wires 15B is inserted into each guide pipe 17B to be movable back and forth in the guide pipe 17B. A tip end of the guide pipe 17B is fixed to the inner surface of the bending portion 12 at a rear end of the bending portion 12. A proximal end of the guide pipe 17B is situated in the operation unit 2.

Fig. 6 is a cross section of the bending portion 12 cut along a plane perpendicular to the axis line of the bending portion 12 (i.e., a cross section along a line VI-VI in Fig. 2). As shown in Fig. 6, four shape-holding wires 15B are arranged about the axis line to have substantially the same angular intervals of 90 degrees. That is, the shape-holding wires 15B are arranged to have angular positions shifted by a certain angle from the angular positions of the shape-holding wires 15A.

The bending portion 12 formed as described above can be deformed freely in response to change of the shape of an insertion tube of a flexible endoscope inserted into the endoscope guiding channel 4 because the four shape-holding wires 15B are not fixed in the bending portion 12 excepting the tip ends thereof and thereby the shape-holding wires 15B are movable in the axial direction. In the state where all of the proximal ends of the shape-holding wires 15B and the guide pipes 17B are fixed in the operation unit 2, the bending portion 12 becomes unable to bend. That is, the shape of the bending portion 12 is fixed.

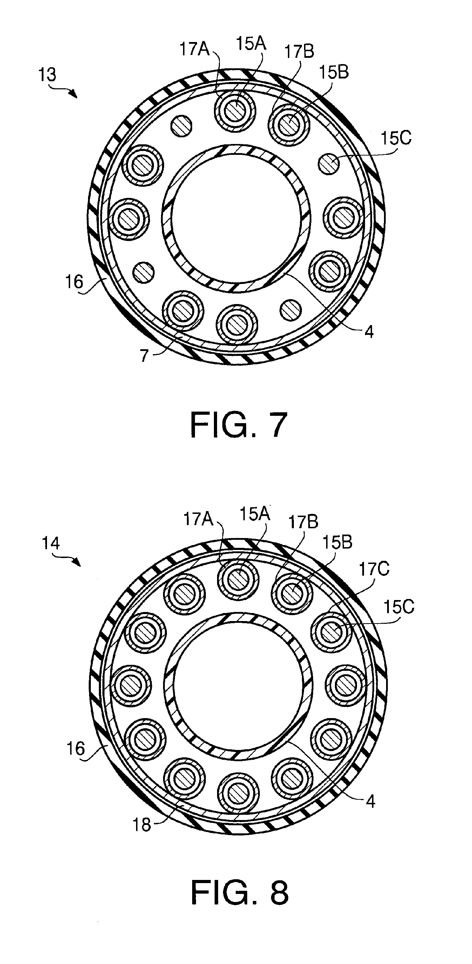

The bending portion 13 has substantially the same structure as that of the bending portions 11 and 12. Fig. 7 is a cross section of the bending portion 13 cut along a plane perpendicular to the axis line (i.e., a cross section cut along a line VII-VII in Fig. 2). As shown in Fig. 7, four shape-holding wires 15C are arranged about the axis line to have substantially the same angular intervals of 90 degrees. That is, the shape-holding wires 15C are arranged to have angular positions shifted by a certain angle from the angular positions of the shape-holding wires 15B (or 15A).

As shown in Fig. 2, a tip end of each shape-holding wire 15C is fixed to the inner surface of the foremost joint ring 7 in the holding portion 13. Flexible guide pipes 17C are provided in the bending portion 13 to be aligned with the shape-holding wires 15C, respectively. Each of shape-holding wires 15C is inserted into each guide pipe 17C to be movable back and forth in the guide pipe 17C. A tip end of the guide pipe 17C is fixed to the inner surface of the bending portion 13 at a rear end of the bending portion 13. A proximal end of the guide pipe 17C is situated in the operation unit 2. That is, each guide pipe 17C is provided such that only the tip end thereof is fixed in the flexible insertion tube 1. It is noted that if the flexible insertion tube 1 is configured not to have the flexible tube part 14, the guide pipe 17C may be omitted.

The bending portion 13 formed as described above can be deformed freely in response to change of the shape of an insertion tube of a flexible endoscope inserted into the endoscope guiding channel 4 because the four shape-holding wires 15C are not fixed in the bending portion 13 excepting the tip ends thereof and thereby the shape-holding wires 15C are movable in the axial direction. In the state where all of the proximal ends of the shape-holding wires 15C and the guide pipes 17C are fixed in the operation unit 2, the bending portion 13 becomes unable to bend. That is, the shape of the bending portion 13 is fixed.

As described above, the bending portions 11-13 connected in series are configured such that one of the bending portions 11-13 can be deformed freely regardless of deformed shapes and deformed directions of the other ones of the bending portions 11-13. Therefore, it is possible to precisely trace along a cavity (e.g., a body cavity in an organ) winding in three dimensions by controlling conditions of the bending portions 11-13. In addition, each of the guide pipes 17A, 17B and 17C that guide the shape-holding wires 15A, 15B and 15C are arranged in the flexible insertion tube 1 such that a part of each guide pipe (17A, 17B, 17C) expecting the tip end thereof is allowed to move freely in the axial direction. Such a configuration provides, for each of the bending portions 11-13 and the flexible tube part 14, a property of being deformed freely while producing the extremely small amount of friction.

By locking the proximal ends of the shape-holding wires 15A, 15B and 15C and the proximal ends of the guide pipes 17A, 17B and 17C in the operation unit 2, it is possible to keep the bent shape of the bending portions 11-13. That is, even if the bending portions 11-13 are respectively bent in different planes, it is possible to keep the bent shapes of the bending portions 11-13 stably while preventing the bending portions 11-13 from being affected with respect to each other. It is also possible to keep the bending portions 11-13 in a straight shape.

Each of the bending portions 11-13 is hard to keep by itself the shape deformed in three dimensions. That is, each of the bending portions 11-13 exhibits a bent shape which lies in a single plane. However, the entire configuration of the bending portions 11-13 coupled in series exhibits a property that the bending portions 11-13 are able to fix the deformed shape provided by exactly tracing a shape deformed in three dimensions.

The flexible tube part 14 has a spiral tube 18 as a framework. The spiral tube 18 is formed by winding stainless steel in a spiral shape to have a certain outer diameter. For example, the spiral tube 18 may be formed such that two spiral tubes having different winding directions are overlapped with each other.

An outer surface of the spiral tube 18 is covered with a mesh tube to prevent extension and twist of the flexible tube part 14. The outermost layer of the flexible tube part 14 is formed of the outer sheath tube 16. It is noted that an arrangement in which barrel-shaped members (similar to the joint rings 7) are coupled may be employed for the flexible tube part 14 in place of the spiral tube 18.

Fig. 8 illustrates a cross section of the flexible tube part 14 cut along a line VIII-VIII in Fig. 2. As shown in Fig. 8, in the flexible tube part 14, the endoscope guiding channel 4 is positioned in the vicinity of the axis line, and twelve shape-holding wires 12A, 12B and 12C are located around the flexible tube part 14 while being inserted into the guide pipes 17A, 17B and 17C, respectively.

The flexible tube part 14 formed as above can be deformed in response to change of the shape of an insertion tube of a flexible endoscope inserted into the endoscope guiding channel 4. That is, the flexible tube part 14 can be deformed freely regardless of the deformed shapes and deformed directions of the bending portions 11-13, although the flexible tube part 14 does not have the ability to fix its shape.

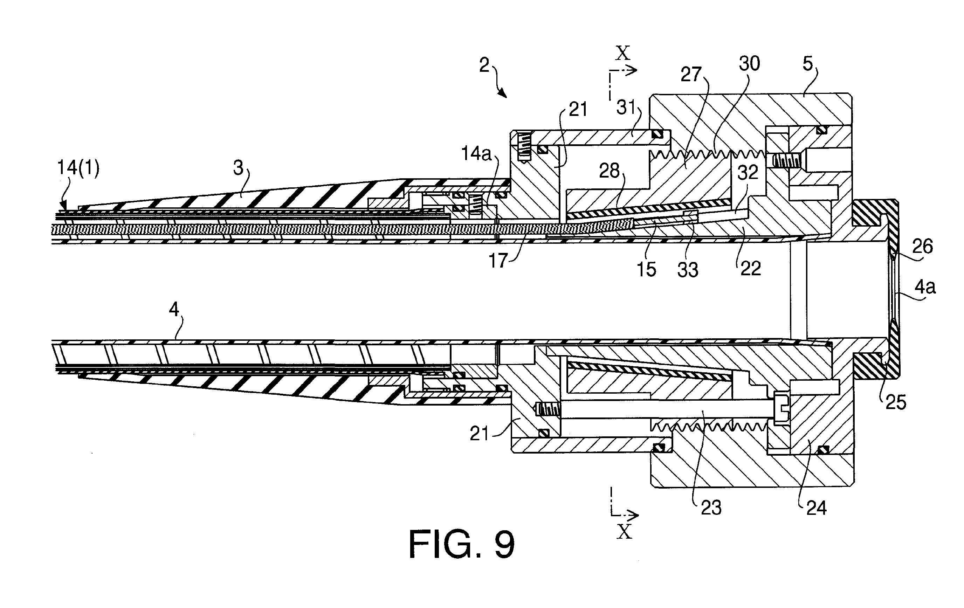

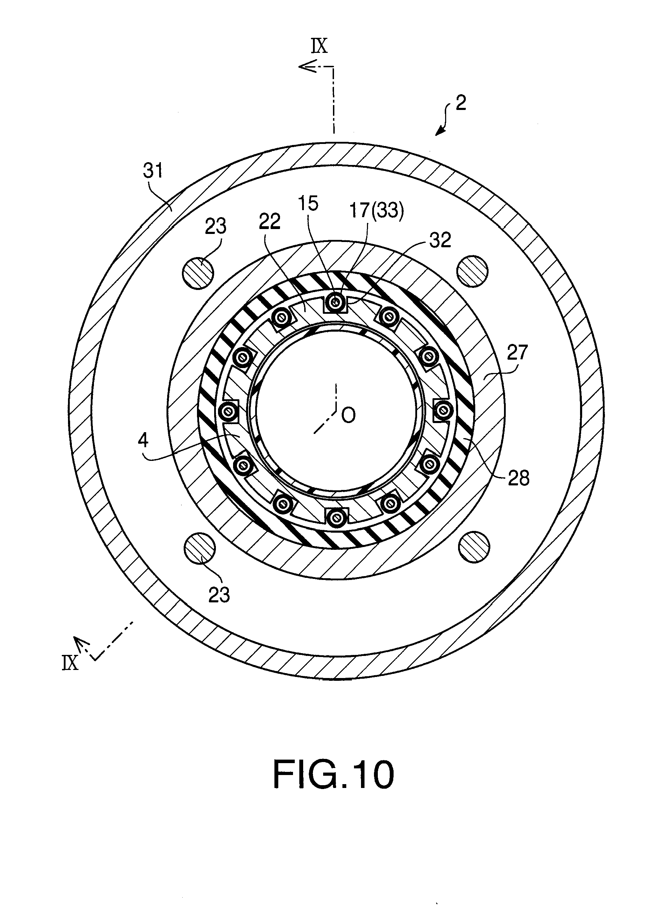

Figs. 9 and 10 illustrate an internal structure of the operation unit 2. Fig. 9 is a side cross section of the operation unit 2, and Fig. 10 is a cross section of the operation unit 2 cut along a line X-X in Fig. 9. It is noted that in Fig. 9 different cross sectional parts (corresponding to IX-O-IX lines in Fig. 10) are illustrated in the upper part and the lower part thereof, respectively.

As shown in Fig. 9, an insertion tube connector 21 and a fixing base 22 are fixed together by four guide shafts 23. To the insertion tube connector 21, a mouthpiece 14a of the flexible tube part 14 is screwed. The fixing base 22 is located such that the flexible tube forming the endoscope guiding channel 4 is inserted in the inside of the fixing base 22 along the axis line of the flexible insertion tube 1. The guide shafts 23 are arranged in parallel with the axis line and are positioned about the axis line of the endoscope guiding channel 4 to have substantially the same angular intervals of 90 degrees. The reference symbol "3" in Fig. 9 is the above described safety stopper.

The proximal end of the flexible tube forming the endoscope guiding channel 4 is nipped between the fixing base 22 and a tube fixing member 24 fixed to the fixing base 22 by screws to achieve a watertight property. An insertion hole sleeve 25 is formed to project backward from the tube fixing member 24. The insertion hole sleeve 25 forms a proximal opening 4a of the endoscope guiding channel 4. It should be noted that each of the fixing base 22, the tube fixing member 24 and other components in the operation unit 2 may be formed of a plurality of parts, for example, for the purpose of enhancing manufacturability.

To the insertion hole sleeve 25, a ring-shaped sealing valve 26 made of elastic material (e.g., rubber) is detachably attached. An endoscope insertion hole having a diameter slightly smaller than the outer diameter of an insertion tube of a flexible endoscope is formed in the sealing valve 26. Therefore, in the state where an insertion tube of a flexible endoscope is inserted in the endoscope guiding channel 4, the sealing valve 26 is pressed against the outer surface of the insertion tube of the flexible endoscope. With this configuration, the sealing valve 26 serves to seal the endoscope guiding channel 4 such that air or liquid is prevented from invading into the endoscope guiding channel 4 through gaps between the sealing valve 26 and the outer surface of the flexible insertion tube of the endoscope.

The outer surface of the fixing base 22 is tapered toward the front end thereof. That is, the outer diameter of the fixing base 22 becomes smaller at a point closer to the front end to have a cone shape. A tapered cylinder 27 having a tapered hole facing the cone-shaped outer surface of the fixing base 22 is mounted in the operation unit 2 to be slidable back and forth along the four guide shafts 23.

The inner surface of the tapered cylinder 27 facing the cone-shaped outer surface of the fixing base 22 is formed of a pressing member 28 having elasticity. More specifically, the tapered cylinder 27 is formed by attaching the pressing member 28 made of material having elasticity and a certain degree of rigidity (e.g. rubber) to the inner surface of the tapered cylinder 27 made of metal so that the tapered cylinder 27 and the pressing member 28 form an integrated structure.

A male thread formed on the outer surface of the tapered cylinder 27 is engaged with a female thread formed on an inner surface of an operation ring 5. A reference symbol "30" in Fig. 9 represents the engagement between the tapered cylinder 27 and the operation ring 5. The operation ring 5 is mounted in the operation unit 2 to be rotatable about the axis line, and the movement of the operation ring 5 in the axial direction is restricted by a cover cylinder 31 which is fixed to the insertion tube connector 21 by screws. With this configuration, by rotating the cover cylinder 31 about the axis line, the tapered cylinder 27 engaged with the cover cylinder 31 also moves in the axial direction along the guide shaft 23.

On the cone-shaped outer surface of the fixing base 22, guide grooves 32 are formed. Each of the guide grooves 32 has a constant width and a constant depth, and is formed in straight line along a slanting surface of the fixing base. In the guide grooves 32, the proximal end of the shape-holding wire 15 (15A, 15B or 15C) and the proximal end of the guide pipe 17 (17A, 17B or 17C) are guided. In the following, frequently, the shape-holding wires 15A, 15B and 15C are generically called the shape holding wire 15, and the guide pipes 17A, 17B and 17C are generically called the guide pipe 17.

At the proximal end of each shape-holding wire 15 (or in the vicinity of the proximal end of each shape-holding wire 15), a rigid proximal mouthpiece 33 is fixed. The proximal mouthpiece 33 has the outer diameter equal to the outer diameter of the guide pipe 17. As shown in Fig. 10, actually, the guide grooves 32, the number of which is equal to the number of shape-holding wires 15, are arranged on the outer surface of the fixing base 22 to have the same angular intervals. In this embodiment, twelve guide grooves 32 are formed on the outer surface of the fixing base 22.

Each guide groove 32 is formed to have a width slightly larger than the outer diameter of the guide pipe 17 and the proximal mouthpiece 33 so that the proximal end of the guide pipe 17 and the proximal mouthpiece 33 are guided smoothly in each guide groove 32.

The depth of each guide groove 32 is slightly smaller than the outer diameter of the proximal end of the guide pipe 17 and the proximal mouthpiece 33. For example, the depth of each guide groove 32 is approximately a half to three-fourths of the outer diameter of the proximal end of the guide pipe 17 and the proximal mouthpiece 33. With this configuration, the proximal end of the guide pipe 17 and the proximal mouthpiece 33 are loosely engaged with the guide groove 32 in the condition where the top of each of the proximal end of the guide pipe 17 and the proximal mouthpieces 33 protrudes slightly from the cone-shaped outer surface of the fixing base 22.

In the operation unit 2, sealing O-rings may be provided at a boundary between adjacent components to keep an inner space at a watertight level. The inner space is a space formed continuously from the tip end of the flexible insertion tube 1 to the operation unit 2, and accommodates the components including the shape-holding wires 15.

In an environment where an endoscope device after an endoscopic examination is placed in a sterilization device for sterilization (i.e., the endoscope device is placed in an vacuum), there is a possibility that the flexible outer sheath tube 16 covering the flexible insertion tube 1 expands. Considering such a phenomenon, the operation unit 2 may be provided with a check valve which opens to connect the inner space with the outside when the pressure of the inner space becomes higher than the pressure of the outside by a certain value. Additionally, the operation unit 2 may be provided with an open valve which can be operated externally to keep the connected state between the inner space and the outside.

In the endoscope guiding tube device 100 configured as described above, all of the twelve shape-holding wires 15 and the guide pipes 17 are arranged in the flexible insertion tube 1 such that only tip ends of the shape-holding wires 15 and the guide pipes 17 are fixed in the flexible insertion tube 1 and the other parts of the shape-holding wires 15 and the guide pipes 17 are movable freely in the axial direction. It is noted that, in the operation unit 2, a driving mechanism for pressing or withdrawing the proximal ends of the shape-holding wires 15 and the guide pipes 17 in the axial direction is not provided.

As shown in Fig. 9, the guide pipe 17 and the shape-holding wire 15 are arranged such that the proximal end of the guide pipe 17 and the proximal end of the shape-holding wire 15 to which the proximal mouthpieces 33 is attached are movable freely back and forth in the guide groove 32 formed on the outer circumferential part of the fixing base 22. Further, the pressing member 28 is arranged to face the outer circumferential part of the fixing base 22 while making a minute gap with respect to the outer circumferential part of the fixing base 22.

When the flexible insertion tube 1 is bent at one or more positions along the bending portions 11-13 and the flexible tube part 14, each of the proximal ends of the shape-holding wires 15 and the guide pipes 17 moves freely in the axial direction in accordance with the degree of bending of each bent portion and the position of each bent portion along the flexible insertion tube 1. Consequently, the bending portions 11-13 and the flexible tube part 14 are capable of deforming passively in response to change of the shape of an insertion tube of a flexible endoscope inserted into the endoscope guiding channel 4.

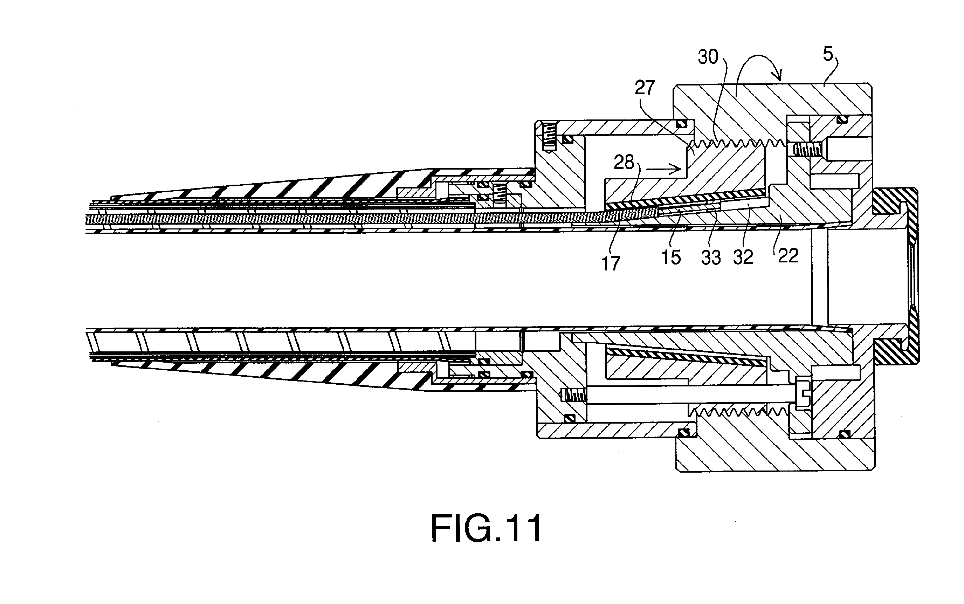

As shown in Fig. 11, when the tapered cylinder 27 is moved backward (i.e., in a rightward direction on Fig. 11) by rotating the operation ring 5, the pressing member 28 attached on the inner surface of the tapered cylinder 27 is pressed against the guide pipe 17 and the proximal mouthpiece 33. Consequently, the proximal end of the guide pipe 17 and the proximal mouthpiece 33 are locked in the groove 32 while being sandwiched between the tapered cylinder 27 and the fixing base 22.

It is noted that the pressing member 28 having flexibility is provided on the inner surface of the tapered cylinder 27. Such a configuration makes it possible to simultaneously lock all of the twelve the proximal ends of the guide pipes 17 and the proximal mouthpieces 33 between the pressing member 28 and the fixing base 22 even if the components including the guide pipes 17 and the proximal mouthpieces 33 in the endoscope guiding tube device 100 have variations in size.

Therefore, each of the bending portions 11-13 is able to maintain the shape defined immediately before the operation for locking the bending portions 11-13. Since each bending portion (11, 12 or 13) is able to deform independently from deformation of the others of the bending portions 11-13, the bending portions 11-13 are able to maintain a complicated shape even if an external forth acts on the fixed shape of the bending portions 11-13.

When the locked state of the proximal ends of the guide pipes 17 and the proximal mouthpieces 33 is released by rotating the operation ring 5 to move the tapered cylinder 27 frontward (i.e., in a leftward direction on Fig. 11), the proximal ends of the guide pipes 17 and the proximal mouthpieces 33 become able to move freely in the axial direction as shown in Fig. 9. That is, the bending portions 11-13 return to the state where all of the bending portions 11-13 deform passively in response to the shape of the insertion tube of the endoscope in the endoscope guiding channel 4.

As described above, in this embodiment, a wire locking mechanism for locking together the proximal ends of all of the shape-holding wires 15 and for releasing the locked state of the proximal ends of the shape-holding wires 15, and a guide locking mechanism for locking together the proximal ends of all of the guide pipes 17 and for releasing the locked state of the proximal ends of all of the guide pipes 17 are achieved as a single locking mechanism. That is, the locked and released states of the shape-holding wires 15 and the guide pipes 17 are controlled through a single locking mechanism.

However, in another embodiment, an endoscope guiding tube device may be provided with separate locking mechanisms: a first one corresponding to the wire locking mechanism; and a second one corresponding to the guide locking mechanism. In this case, each shape-holding wire 15 is not required to have the proximal mouthpiece 33. When the shape-holding wire 15 is configured not to have the proximal mouthpiece 33, the proximal end portion of the shape-holding wire 15 is pressed directly by the pressing member 28 of the tapered cylinder 27.

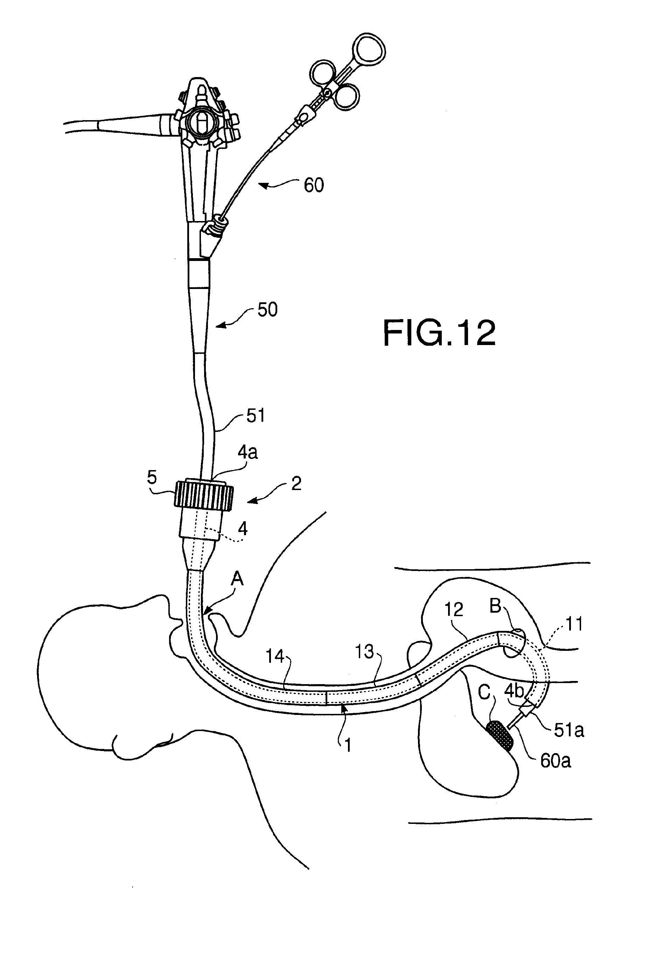

Fig. 12 illustrates a situation where the endoscope guiding tube device 100 is used for endoscopic treatment. For the treatment, a tip end 51a of an insertion unit 51 of a flexible endoscope 50 is inserted into the endoscope guiding channel 4 so that the tip end 51a of the insertion unit 51 protrudes from the tip end of the flexible insertion tube 1. In this state (i.e., in the state where the bending portions 11-13 are able to deform freely in response to the change in shape of the flexible insertion unit 51), the flexible insertion tube 1 is inserted, from a mouth A of a patient, into a stomach through an esophagus.

Then an operator operates the flexible endoscope 50 so that the tip end 51a exits from the stomach through a hole B formed in a wall of the stomach and the tip end 51a reaches a target (a cholecyst C). In the state where the tip end 51a of the flexible insertion unit 51 faces the target (the cholecyst C), the operator operates the operation ring 5 to fix the deformed shapes of the bending portions 11-13.

Since the deformed shape of each of the bending portions 11-13 can be fixed by operation the operation ring 5, it is possible to smoothly and safely conduct the treatment for the cholecyst C while using a treatment instrument 60 inserted into a treatment instrument channel of the flexible endoscope 50. In the state where the deformed shape of each of the bending portions 11-13 is fixed, it is possible to quickly replace the endoscope 50 with another one. In Fig. 12, a reference symbol 60a represents a tip end of the treatment instrument 60.

Although the present invention has been described in considerable detail with reference to certain preferred embodiments thereof, other embodiments are possible.

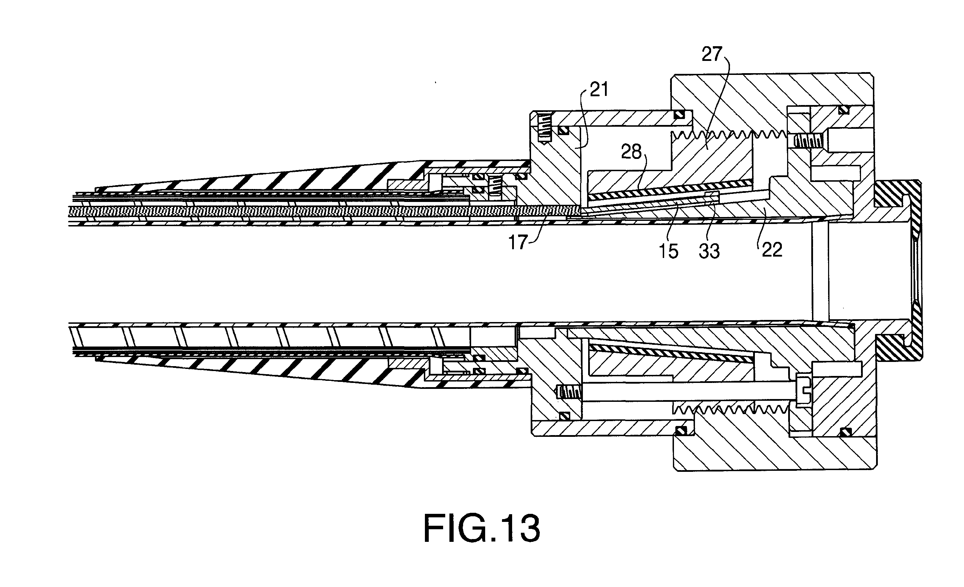

For example, as shown in Fig. 13, the proximal end of each guide pipe 17 may be fixed to one of the components in the operation unit 2 (e.g., the insertion tube connector 21) without allowing the guide pipe 17 to move freely in the axial direction. Although in this case the guide pipe 17 deforms in response to the bending motion of the flexible insertion tube 1, the flexible insertion tube 1 is able to retain the function of fixing the shape of each of the bending portions 11-13.

In the above described embodiment, each of the wire locking mechanism and the guide locking mechanism is configured such that a tapered hole (the tapered cylinder 27) is moved along the axial direction. However, various types of mechanisms for locking or releasing the end portions of the shape-holding wires 17 and the guide pipes 17 may be employed.

In the above described embodiment, all of the proximal ends of the shape-holding wires 15A, 15B and 15C and the proximal ends of the guide pipes 17A, 17B and 17C are locked in the operation unit 2 to keep the bent shape of the bending portions 11-13. However, substantially the same advantages (i.e., to keep the bent shape of the bending portions 11-13) can also be achieved by only locking the proximal ends of the shape holding wires 15A, 15B and 15C.

In the above described embodiment, the flexible insertion tube 1 is formed by connecting three bending portions 11-13 in series. The number of bending portions is determined by achieving a balance between complexity of the endoscope guiding tube device and functionality of the endoscope guiding tube device. However, the flexible insertion tube may be provided with four or more bending portions.

## Claims

1. An endoscope guiding tube device, comprising:
a flexible insertion tube;
an endoscope guiding channel that is arranged in the flexible insertion tube along a length of the flexible insertion tube such that an insertion unit of an flexible endoscope can be inserted into the endoscope guiding channel;
at least one bending portion that forms the flexible insertion tube and has a framework including a plurality of barrel-shaped joint rings coupled to each other in series such that adjacent ones of the plurality of barrel-shaped joint rings are rotatable with respect to each other about a joint shaft provided between the adjacent ones of the plurality of barrel-shaped joint rings, wherein the framework is deformable freely in all directions, and wherein the at least one bending portion is deformable passively in accordance with change in shape of the insertion unit of the flexible endoscope inserted into the endoscope guiding channel;
a plurality of shape-holding wires of which tip ends are attached to a tip portion of the at least one bending portion; and
an operation unit provided at a proximal side of the flexible insertion tube and is configured such that proximal ends of the plurality of shape-holding wires are arranged in the operation unit to be movable freely in an axial direction of the flexible insertion tube, the operation unit including a wire locking mechanism having a function of locking together all of the proximal ends of the plurality of shape-holding wires and a function of releasing a locked state of all of the proximal ends of the plurality of shape-holding wires.

2. The endoscope guiding tube device according to claim 1, wherein the plurality of shape-holding wires comprises at least three shape-holding wires.

3. The endoscope guiding tube device according to claim 2,
wherein the at least three shape-holding wires are arranged about an axis line of the flexible insertion tube such that adjacent ones of the at least three shape-holding wires do not have an angular interval larger than or equal to 180 degrees.

4. The endoscope guiding tube device according to any preceding claim,
wherein the endoscope guiding channel has a flexible tube arranged near an axis line of the flexible insertion tube.

5. The endoscope guiding tube device according to any preceding claim,
further comprising a sealing member provided at a proximal end of the endoscope guiding channel to seal a gap formed between an edge of an opening of the proximal end of the endoscope guiding channel and the insertion unit of the endoscope inserted into the endoscope guiding channel.

6. The endoscope guiding tube device according to any preceding claim,
further comprising an outer sheath that has an elasticity and covers the at least one bending portion.

7. The endoscope guiding tube device according to any preceding claim,
wherein:
the at least one bending portion is provided on a front side of the flexible insertion tube; and
a part of the flexible insertion tube other than the at least one bending portion is formed as a flexible tube part that is deformable passively in accordance with change in shape of the insertion unit of the flexible endoscope inserted into the flexible insertion tube, the flexible part being configured not to have a property of folding a shape thereof.

8. The endoscope guiding tube device according to any preceding claim,
wherein the wire locking mechanism comprises:
a fixing base having a cone-shaped outer surface on which the proximal ends of the plurality of shape-holding wires move;
a tapered cylinder that is arranged to be movable freely in the axial direction of the flexible insertion tube and has a tapered hole formed therein to form an inner surface facing the cone-shaped outer surface of the fixing base; and
an operating member that moves the tapered cylinder in the axial direction,
wherein:
when the tapered cylinder is moved to a rear side by the operating member, the proximal ends of all of the plurality of shape-holding wires are sandwiched between the tapered cylinder and the fixing base and thereby the proximal ends of all of the plurality of shape-holding wires are locked; and
when the tapered cylinder is moved to a front side by the operating member, the proximal ends of all of the plurality of shape-holding wires become able to be movable in the axial direction, and thereby the at least one bending portion becomes able to be deformable passively in accordance with change in shape of the insertion unit of the flexible endoscope inserted into the endoscope guiding channel.

9. The endoscope guiding tube device according to claim 8, wherein an elastic member is provided on the inner surface of the tapered cylinder.

10. The endoscope guiding tube device according to claim 8 or 9,
wherein:
each of the plurality of shape-holding wires is provided with a proximal mouthpiece at a proximal end portion thereof; and
the proximal mouthpiece is locked between the tapered cylinder and the fixing base when the tapered cylinder is moved to the rear side.

11. The endoscope guiding tube device according to claim 10,
wherein:
a guiding groove is formed on the cone-shaped outer surface of the fixing base;
the proximal mouthpiece of each of the plurality of shape-holding wires is guided, along the guiding grove, to be movable in the axial direction; and
a depth of the guiding groove is smaller than a diameter of the proximal mouthpiece of each of the plurality of shape-holding wires.

12. The endoscope guiding tube device according to any of claims 8 to 11,
wherein a plurality of guide shafts is provided in the operation unit to guide the tapered cylinder to be movable in the axial direction.

13. The endoscope guiding tube device according to claim 12,
wherein:
the operation member includes an operation ring mounted in the operation unit to be rotatable about an axis line of the flexible insertion tube, a female thread being formed on the operation ring;
the tapered cylinder is provided with a male thread such that the male thread engages with the female thread of the operation ring; and
the tapered cylinder moves in the axial direction by rotating the operation ring about the axis line.

14. The endoscope guiding tube device according to claim 1,
further comprising a plurality of flexible guide pipes of which tip ends are attached to a rear portion of the at least one bending portion, the plurality of shape-holding wires being respectively inserted into the plurality of flexible guide pipes to be movable in the axial direction,
wherein:
proximal ends of the plurality of flexible guide pipes are arranged in the operation unit to be movable in the axial direction; and
the operation unit comprises a guide locking mechanism having a function of locking together all of the proximal ends of the plurality of flexible guide pipes and a function of releasing a locked state of all of the proximal ends of the plurality of flexible guide pipes.

15. The endoscope guiding tube device according to claim 14,
wherein each of the plurality of flexible guide pipes is formed of a coil pipe formed by tightly winding a metal wire to have a predetermined diameter.

16. The endoscope guiding tube device according to claim 14 or 15,
wherein the wire locking mechanism and the guide locking mechanism are formed as a single locking mechanism.

17. The endoscope guiding tube device according to claim 16,
wherein all of the proximal ends of the plurality of shape-holding wires and the plurality of flexible guide pipes are locked simultaneously by the single locking mechanism, and the locked state of all of the proximal ends of the plurality of shape-holding wires and the plurality of flexible guide pipes are released simultaneously by the single locking mechanism.

18. The endoscope guiding tube device according to any of claims 14 to 17, wherein each of the plurality of flexible guide pipes is fixed in the operation unit.

19. The endoscope guiding tube device according to any of claims 14 to 18,
wherein:
the at least one bending portion comprises a plurality of bending portions coupled to each other in series;
the tip ends of the plurality of flexible guide pipes are attached to rear portions of the plurality of bending portions, respectively; and
the guide locking mechanism is able to lock together all of the proximal ends of the plurality of flexible guide pipes and is able to release the locked state of all of the proximal ends of the plurality of flexible guide pipes.

20. The endoscope guiding tube device according to any of claims 14 to 19,
wherein the guide locking mechanism comprises:
a fixing base having a cone-shaped outer surface on which the proximal ends of the plurality of shape-holding wires move;
a tapered cylinder that is arranged to be movable freely in the axial direction of the flexible insertion tube and has a tapered hole formed therein to form an inner surface facing the cone-shaped outer surface of the fixing base; and
an operating member that moves the tapered cylinder in the axial direction,
wherein:
when the tapered cylinder is moved to a rear side by the operating member, the proximal ends of all of the plurality of guide pipes are sandwiched between the tapered cylinder and the fixing base and thereby the proximal ends of the plurality of guide pipes are locked; and
when the tapered cylinder is moved to a front side by the operating member, the proximal ends of the plurality of guide pipes become able to be movable in the axial direction.

21. The endoscope guiding tube device according to claim 20,
wherein:
each of the plurality of shape-holding wires is provided with a proximal mouthpiece at a proximal end portion thereof; and
the proximal mouthpiece is locked between the tapered cylinder and the fixing base when the tapered cylinder is moved to the rear side.

22. The endoscope guiding tube device according to claim 21,
wherein:
the proximal mouthpiece has an outer diameter equal to an outer diameter of each of the proximal ends of the plurality of flexible guide pipes; and
proximal mouthpieces of the plurality of shape-holding wires and the proximal ends of the plurality of flexible guide pipes are locked simultaneously between the tapered cylinder and the fixing base when the tapered cylinder is moved to the rear side.

23. The endoscope guiding tube device according to claim 22,
wherein:
a guiding groove is formed on the cone-shaped outer surface of the fixing base;
the proximal end of each of the plurality of flexible guide pipes is guided, along the guiding groove, to be movable in the axial direction; and
a depth of the guiding groove is smaller than a diameter of the proximal end of each of the plurality of flexible guide pipes.

24. The endoscope guiding tube device according to claim 1,
wherein the at least one bending portion comprises at least three bending portions coupled to each other in series.
